(19) Europäisches Patentamt
European Patent Office
Office européen des brevets

(11) Veröffentlichungsnummer: **0 047 928**
**B1**

(12) # EUROPÄISCHE PATENTSCHRIFT

(45) Veröffentlichungstag der Patentschrift: **21.09.83**

(51) Int. Cl.³: **C 07 J 19/00**, A 61 K 31/705

(21) Anmeldenummer: **81106938.4**

(22) Anmeldetag: **04.09.81**

(54) **Neue Derivate von Cardenolid-bis-digitoxosiden, Verfahren zu ihrer Herstellung und ihre Verwendung als Arzneimittel.**

(30) Priorität: **13.09.80 DE 3034658**

(43) Veröffentlichungstag der Anmeldung: **24.03.82 Patentblatt 82/12**

(45) Bekanntmachung des Hinweises auf die Patenterteilung: **21.09.83 Patentblatt 83/38**

(84) Benannte Vertragsstaaten: **AT BE CH DE FR GB IT LI LU NL SE**

(56) Entgegenhaltungen:
**DE-A-2 013 221**

**CHEMICAL ABSTRACTS, Band 83, Nr. 25, 22. Dezember 1975, Seite 8, Zusammenfassung Nr. 201744h Columbus, Ohio, U.S.A., A. YODA et al.: "Structure-activity relations of cardiotonic steroids for the inhibition of sodium- and potassium-dependent adenosine triphosphatase. V. Dissociation rate constants of digitoxin acetates"**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Schaumann, Wolfgang, Prof. Dr., Mönchhofstrasse 58, D-6900 Heidelberg (DE)**
Erfinder: **Kaiser, Fritz, Dr., Hans-Holbein-Strasse 10, D-6840 Lampertheim (DE)**
Erfinder: **Voigtländer, Wolfgang, Dr., Haselnussweg 30, D-6940 Weinheim (DE)**
Erfinder: **Hoyer, Edgar, In den alten Wiesen 55, D-6800 Mannheim (DE)**
Erfinder: **Koch, Klaus, Dr., Glücksburger Weg 168, D-6800 Mannheim 31 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Neue Derivate von Cardenolid-bis-digitoxosiden, Verfahren zu ihrer Herstellung und ihre Verwendung als Arzneimittel

Gegenstand der Erfindung sind neue Verbindungen der Formel I

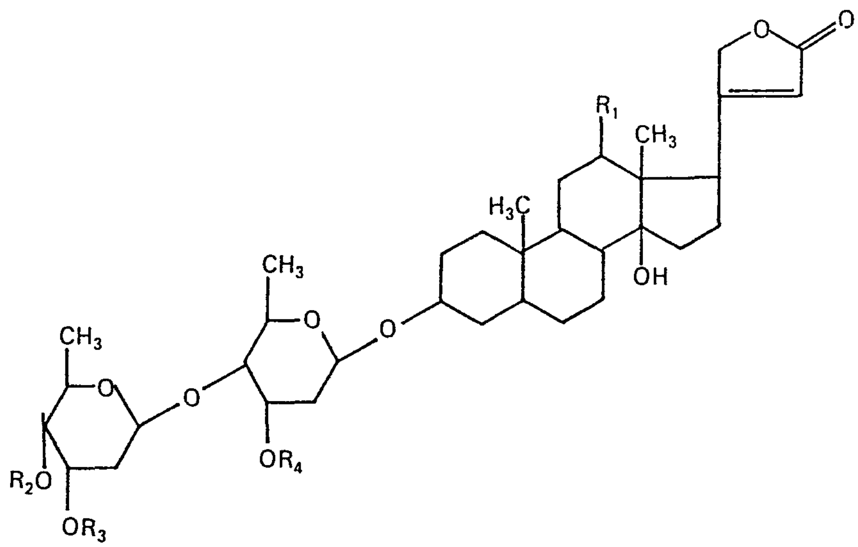

(I)

in der
$R_1$ Wasserstoff, eine Hydroxylgruppe oder einen Acyloxyrest mit 1-3 Kohlenstoffatomen darstellt,
$R_2$, $R_3$ und $R_4$ gleich oder verschieden sind und Wasserstoff, Acylgruppen mit 1-3 Kohlenstoffatomen oder Alkylgruppen mit 1-3 Kohlenstoffatomen bedeuten,
wobei die Verbindung jedoch mindestens eine Alkylgruppe enthält,

und Verfahren zu ihrer Herstellung sowie ihre Verwendung zur Herstellung von Arzneimitteln gegen Herzinsuffizienz.

Die in der Therapie der Herzinsuffizienz hauptsächlich verwendeten Digitalisglykoside Digitoxin und Digoxin mit ihren Derivaten, z.B. Acetyldigoxin, Methyldigoxin, lassen in der Breite ihrer Anwendungsmöglichkeiten noch Wünsche offen.

Digoxin und Derivate werden überwiegend durch die Nieren ausgeschieden und können deshalb bei Patienten mit verringerter Nierenfunktion zu Intoxikationen führen.

Digitoxin wird dagegen extrarenal ausgeschieden, ist aber von den obigen Verbindungen das Glykosid mit der längsten Verweildauer im Organismus, weshalb etwa auftretende Intoxikationen (z.B. bei Überdosierung) nur extrem langsam wieder abklingen können.

Aus der DEA 2013221 sind Steroidglykoside mit 2-Desoxyzuckerresten unter anderem Digitoxigenin-bis-digitoxosid und Digoxigenin-bis-digitoxosid bekannt. Verbindungen dieser Art zeigen eine extrem kurze Verweilzeit, weil sie über Urin und Fäces rasch ausgeschieden werden.

Es wurde nun gefunden, dass die erfindungsgemässen Derivate der Bis-digitoxoside eine ideale Mittelstellung einnehmen, indem sie erheblich schneller als Digitoxin - in der Grössenordnung wie Digoxin - aber langsamer als die zu flüchtig wirkenden unsubstituierten Bis-digitoxoside ausgeschieden werden. Vor allem verläuft ihre Elimination aus dem Organismus weit überwiegend extrarenal, wodurch sie für die Therapie der Herzinsuffizienz bei gleichzeitiger verminderter Nierenfunktion hervorragend geeignet sind.

Die neuen Cardenolid-bis-digitoxoside können wie folgt hergestellt werden:

a) Verbindungen der Formel II

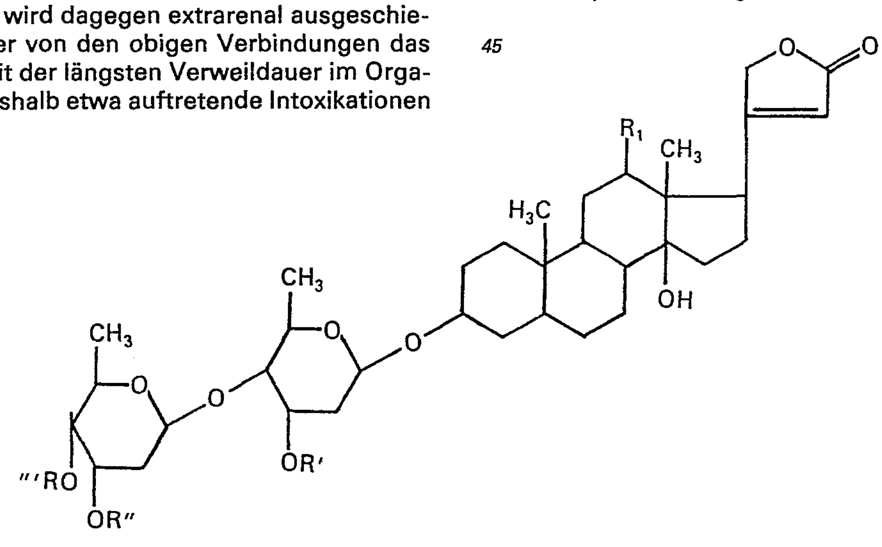

(II)

in der
$R_1$ Wasserstoff oder eine Hydroxylgruppe oder eine Acyloxygruppe,
R′ Wasserstoff, eine Acylgruppe oder eine Alkylgruppe und
R″ und R‴ Wasserstoff, eine Alkylgruppe, eine Acylgruppe oder zusammen ein cyclisches Acetal oder Ketal bedeuten, wobei zumindest eine der Gruppen R′, R″ und R‴ Wasserstoff ist,
werden in an sich bekannter Weise mit einem geeigneten Acylierungs- oder Alkylierungsmittel ein- oder mehrfach acyliert oder alkyliert, worauf gegebenenfalls eine oder mehrere Acyl-, Acetal- oder Ketalgruppen durch selektive Hydrolyse abgespalten werden, worauf gegebenenfalls eine freie Hydroxylgruppe mit einem weiteren Acylierungs- oder Alkylierungsreagenz acyliert oder alkyliert wird, oder

b) Verbindungen der Formel III

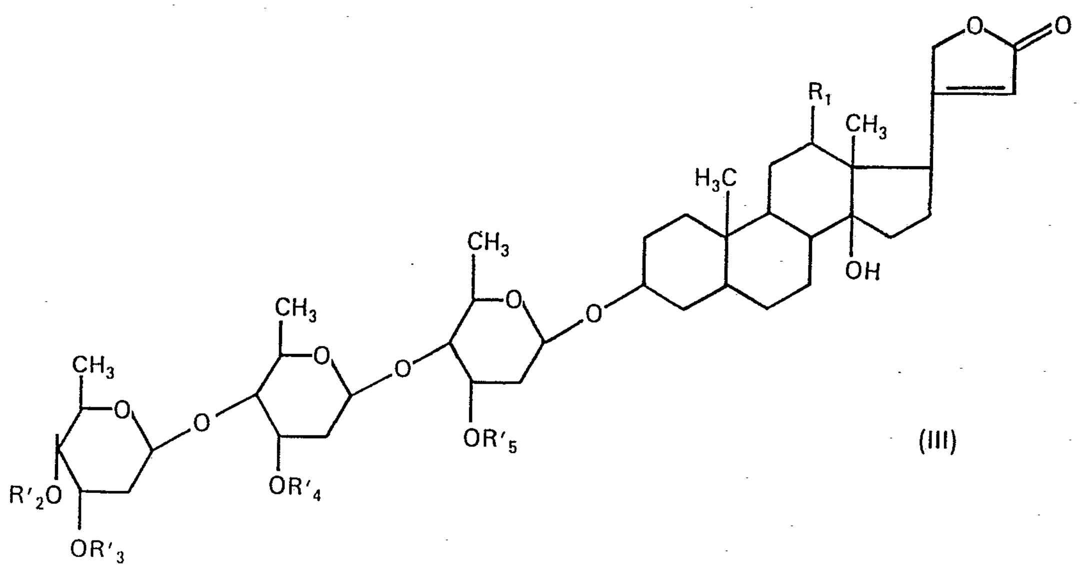

(III)

worin
$R_1$ Wasserstoff oder eine Hydroxylgruppe oder eine Acyloxygruppe,
$R'_2$ und $R'_3$ gemeinsam ein cyclisches Acetal oder Ketal mit 2-5 C-Atomen oder eine Acylgruppe,
$R'_4$ und $R'_5$ Alkylgruppen mit 1-3 C-Atomen oder Acylgruppen bedeuten,
werden durch selektive Hydrolyse der Acyl-, Acetal- bzw. Ketalgruppen $R'_2$ und $R'_3$ und anschliessende oxidative Abspaltung der endständigen Digitoxose umgesetzt, worauf gegebenenfalls eine oder mehrere freie Hydroxylgruppen mit einem geeigneten Acylierungs- und/oder Alkylierungsreagenz in Acyloxy- und/oder Alkyloxygruppen überführt werden.

Zur Alkylierung der Verbindungen der Formeln II und III werden bevorzugt die in DT-AS 1 961 034 und DT-OS 2 233 147 Beispiel 1 beschriebenen Verfahren der Alkylierung mit abgestuften Mengen von Dialkylsulfat bei Gegenwart von Strontiumhydroxyd oder Bariumhydroxyd oder Aluminiumisopropylat und Aluminiumoxid in einem inerten Lösungsmittel angewendet, doch können auch andere übliche Alkylierungsmittel, wie z.B. Alkylhalogenide oder Alkyltosylat, Alkylmesylat (DT-OS 2 734 401) eingesetzt werden.

Die Acylierung der Verbindungen kann z.B. nach Arzneim. Forsch. 15, 481, DBP 1 063 160, Pharm. Bull. 5, 171, 1955 mit abgestuften Mengen von Säureanhydriden in Pyridin oder Dimehtylformamid und gegebenenfalls anschliessender Trennung der Acylierungsprodukte durch multiplikative Verteilung oder chromatographische Methoden erfolgen, wobei bevorzugt in der 10′-Stellung acyliert wird, bei längerer Reaktion jedoch auch die vollständig acylierten Derivate erhalten werden.

Weiterhin kann die Acylierung nach der in DE-AS 2 206 737 beschriebenen Methode mit Dialkylacetamiddialkylacetalen oder mit Orthoestern gemäss DE-AS 2 010 422 in einem inerten Lösungsmittel durchgeführt werden, wobei man bevorzugt in 9′-Stellung acylierte Produkte erhält.

Nach der in DE-OS 2 110 646 beschriebenen Methode, Umsetzung der Verbindung II mit Acylierungsmitteln bei Gegenwart eines teritiären Amins, entstehen überwiegend 9′, 10′-Diacylderivate.

Schliesslich kann man 12-Acyl-digoxigenin-bis-digitoxosid nach der Methode DE-AS 2 126 305 durch Peracylierung und partielle Verseifung erhalten.

Ketalgruppen werden vorzugsweise in schwach saurer Lösung in Wasser oder wässrigen Alkoholen abgespalten.

Acylgruppen, insbesondere die 9′, 10′ – bzw. 15′, 16′-Substituenten, lassen sich vorzugsweise in schwach basischer Lösung, beispielsweise in Wasser/Bicarbonat, abspalten. Als Acylierungsmittel werden wegen ihrer einfachen Handhabung und ihres niedrigen Preises die Säureanhydride bzw. gemischten Säureanhydride bevorzugt, es lassen sich jedoch auch die entsprechenden Säurechloride, Imidazolide oder andere bekannte Acylderivate mit gleichem Erfolg einsetzen.

Die Aufarbeitung und Reinigung der Endprodukte erfolgt nach üblichen Methoden durch multiplikative Verteilung oder chromatographische Methoden und Kristallisation.

Identität und Reinheit der erhaltenen Verbindungen wurden durch Dünnschichtchromatogramme überprüft. Dabei wurden DC-Fertigplatten (Merck Kieselgel 60/F 254 Imprägnierung 20% Formamid in Aceton) eingesetzt und mit Fliessmittel Xylol-Methylethylketon 2:3 + 5% Formamid entwickelt. Die fertigen Chromatogramme wurden mit Trichloressigsäure-Chloramin-Reagens besprüht und die Substanzen durch ihre Fluoreszenzen im langweiligen UV ($\lambda$ = 360 nm) ermittelt. Die Laufstrecken (R) im Chromatogramm wurden jeweils auf einen mitgeführten Standard bezogen. Dabei bedeutet $R_D$ den auf die Laufstrecke von Digitoxigenin-bis-digitoxosid bezogenen R-Wert und $R_{DG}$ den auf die Laufstrecke von Digoxigenin-bis-digitoxosid bezogenen R-Wert.

Die erfindungsgemässen Cardenolidglycoside wirken im gleichen Konzentrationsbereich wie übliche Herzglykoside, wie zum Beispiel Digoxin oder Digitoxin, und können in Einzeldosierungen von 0.050–0.250 mg 1–4 mal täglich appliziert werden. Die Applikation erfolgt vorzugsweise oral, jedoch ist auch eine parenterale Applikation ohne weiteres möglich.

Als orale Darreichungsform werden bevorzugt Tabletten aber auch Steckkapseln und Weichgelatinekapseln verwendet. Zur individuellen Dosierungseinnahme z.B. für Kinder ist die Zubereitung als Liquidum geeignet. Für die Notfall- und Stationärbehandlung erfolgt die Anwendung durch Injektion entsprechender Lösungen.

Zur Herstellung von Tabletten oder Steckkapseln zur oralen Darreichung wird der Wirkstoff mit üblichen Hilfsstoffen, wie Lactose und Stärke homogen gemischt, wobei wegen der geringen Einzeldosierung die Herstellung einer Vormischung bevorzugt wird. Die Wirkstoff-Hilfsstoff-Mischung kann durch Auswahl geeigneter Hilfsstoffe als trockene Pulvermasse oder durch Granulation mit Bindemitteln, wie Stärkekleister oder Polyvinylpyrrolidon als Granulat durch weitere Zumischung üblicher Sprengmittel und Gleitmittel zu Tabletten verpresst abgefüllt werden.

Trägerstoffe für Weichgelatinekapseln können übliche Glycerin-Fettsäureester sein, aber auch Polyethylenglykole als Lösungsmittel für den Wirkstoff. Für eine Liquidum- oder Ampullenform können als Lösungsmittel Ethanol oder mehrwertige Alkohole in Abmischungen verwendet werden.

Beispiel 1

9'-Methyl-digitoxigenin-bis-digitoxosid

4 g Aluminiumisopropylat, in 20 ml Dimethylformamid und 3.2 ml Methyljodid, werden 5 Stunden bei Raumtemperatur gerührt, mit 4 g Digitoxigenin-bis-digitoxosid versetzt und weitere 3 Tage bei Raumtemperatur gerührt. Anschliessend wird mit 320 ml Chloroform verdünnt, über Kieselgur abgesaugt, mit Chloroform nachgewaschen und im Vakuum eingeengt. Der Rückstand wird in 120 ml Methanol und 180 ml 10proz. Essigsäure gelöst, mit Chloroform ausgeschüttelt, die Chloroformphasen mit 5proz. Natriumbicarbonatlösung gewaschen, über Natriumsulfat getrocknet und im Vakuum eingeengt. Das Rohprodukt wird in Essigester gelöst über 200 g Aluminiumoxyd (+5% Wasser) mit Essigester-Chloroform fraktioniert. Die Chloroformfraktion liefert nach Kristallisation aus Aceton-Wasser und Ethanol-Wasser 2.1 g 9'-Methyl-digitoxigenin-bis-digitoxosid.

Schmelzpunkt: 145–148°C $R_D$:1.23

Beispiel 2

10'-Methyl-digitoxigenin-bis-digitoxosid

2 g Digitoxigenin-bis-digitoxosid, in 14 ml Dimethylformamid und 14 ml Toluol gelöst, werden nach Zugabe von 0.68 g Aluminiumoxyd (Merck, nach Brockmann), 1.12 g Strontiumhydroxyd [$Sr(OH)_2 \cdot 8\ H_2O$] und 2.0 ml Dimethylsulfat in 22 ml Toluol 4 Stunden bei Raumtemperatur gerührt. Anschliessend wird mit 40 ml Chloroform verdünnt, über 12 g Kieselgur filtriert, mit 40 ml Chloroform gewaschen, 2.6 ml Morpholin zugegeben, 30 Minuten stehen gelassen, nach Zugabe von 15 ml Wasser 15 Minuten gerührt, die Chloroformphase abgetrennt, mit 10 ml Wasser nochmals 15 Minuten gerührt und die Chloroformphase im Vakuum eingeengt. Das Rohprodukt wird in Essigester gelöst über 10 g Aluminiumoxyd (+5% Wasser) mit Essigester, Chloroform und Chloroform-Methanol 1:1 fraktioniert. Die Chloroformfraktion liefert nach Kristallisation aus Chloroform-Ether und Ethanol-Wasser 1.45 g 10'-Methyl-digitoxigenin-bis-digitoxosid.

Schmelzpunkt: 197–203°C $R_D$:1.24

Beispiel 3

10'-Ethyl-digitoxigenin-bis-digitoxosid

2 g Digitoxigenin-bis-digitoxosid, in 16 ml Dimethylformamid gelöst, werden nach Zugabe von 1.16 g Bariumhydroxyd [$Ba(OH)_2 \cdot 8\ H_2O$], 392 mg Bariumoxyd und 1.68 ml Diethylsulfat unter Überleiten von Stickstoff 6 Stunden bei 30–35°C gerührt. Anschliessend wird mit 80 ml Chloroform verdünnt, über Kieselgur filtriert, mit 40 ml Chloroform gewaschen, 12 ml Morpholin zugegeben, 30 Minuten stehen gelassen, mit 20 ml Wasser ausgeschüttelt und die Chloroformphase im Vakuum eingeengt. Das Rohprodukt wird mit Cyclohexan-Essigester 3:1 über eine Cellulosesäule (mit Formamid imprägniert) getrennt. Die chromatographisch einheitlichen Fraktionen liefern nach Kristallisation aus Essigester 920 mg 10'-Ethyl-digitoxigenin-bis-digitoxosid.

Schmelzpunkt: 213–217°C $R_D$:1.33

Beispiel 4

10'-n-Propyl-digitoxigenin-bis-digitoxosid

2 g Digitoxigenin-bis-digitoxosid, in 16 ml Dimethylformamid gelöst, werden mit 1.16 g Bariumhydroxyd, 392 mg Bariumoxyd und 1.68 ml Di-n-propylsulfat wie unter Beispiel 3 beschrieben umgesetzt und aufgearbeitet. Das Rohprodukt wird mit Heptan-Methylethylketon 3:1 über eine Cellulosesäule (mit Formamid imprägniert) getrennt. Die chromatographisch einheitlichen Fraktionen ergeben nach Kristallisation aus Essigester 820 mg 10′-n-Propyl-digitoxigenin-bis-digitoxosid.

Schmelzpunkt: 228–232°C $R_D$: 1.38

Beispiel 5
9′, 10′-Dimethyl-digitoxigenin-bis-digitoxosid
4 g Digitoxigenin-bis-digitoxosid, in 48 ml Dimethylacetamid gelöst, werden nach Zugabe von 11.2 g Bariumhydroxyd $[Ba(OH)_2 \cdot 8\ H_2O]$ und 14.4 ml Dimethylsulfat 4 Stunden bei Raumtemperatur gerührt. Anschliessend wird mit 300 ml Chloroform verdünnt, über Kieselgur filtriert, mit 300 ml Chloroform gewaschen, 20 ml Morpholin zugegeben, 30 Minuten stehen gelassen, nach Zugabe von 90 ml Wasser 15 Minuten gerührt, die Chloroformphase abgetrennt, mit 60 ml Wasser nochmals 15 Minuten gerührt und die Chloroformphase im Vakuum eingeengt. Das Rohprodukt wird mit Cyclohexan-Essigester 4:1 über eine Cellulosesäule (mit Formamid imprägniert) getrennt. Die chromatographisch einheitlichen Fraktionen liefern nach Kristallisation aus Essigester 1.3 g 9′, 10′-Dimethyl-digitoxigenin-bis-digitoxosid.

Schmelzpunkt: 225–228°C $R_D$: 1.40

Beispiel 6
3′, 9′, 10′-Trimethyl-digitoxigenin-bis-digitoxosid
3 g Digitoxigenin-bis-digitoxosid, in 36 ml Dimethylacetamid gelöst, werden nach Zugabe von 8.4 g Bariumhydroxyd $[Ba(OH)_2 \cdot 8\ H_2O]$ und 10.8 ml Dimethylsulfat wie unter Beispiel 5 beschrieben umgesetzt und aufgearbeitet. Das Rohprodukt wird mit Heptan-Methylethylketon 3:1 über eine Cellulosesäule (mit Formamid imprägniert) getrennt. Die chromatographisch einheitlichen Fraktionen ergeben nach Kristallisation aus Chloroform-Methanol-Petrolether 1.06 g 3′, 9′, 10′-Trimethyl-digitoxigenin-bis- digitoxosid.

Schmelzpunkt: 91–95°C $R_D$: 1.48

Beispiel 7
3′-Methyl-digitoxigenin-bis-digitoxosid
10 g Digitoxigenin-bis-digitoxosid, in 100 ml Chloroform-Methanol 1:1 gelöst, werden nach Zugabe von 260 ml Aceton (wasserfrei), 20 ml 2,2-Dimethoxypropan und 80 mg p-Toluolsulfonsäure 2 Stunden bei Raumtemperatur gerührt, mit 400 ml 5proz. Natriumbicarbonatlösung verdünnt und mit Chloroform ausgeschüttelt. Die Chloroformphase wird nach Waschen mit Wasser und Trocknen über Natriumsulfat im Vakuum eingeengt und nach Lösen in 400 ml Tetrachlorkohlenstoff-Chloroform 4:1 über 100 g Aluminiumoxyd (Merck, nach Brockmann) fraktioniert. Die eingeengte Tetrachlorkohlenstoff-Chloroform-Fraktion ergab nach Kristallisation aus Ether 6.1 g 9′, 10′-Isopropyliden-digitoxigenin-bis-digitoxosid.

6 g 9′, 10′-Isopropyliden-digitoxigenin-bis-digitoxosid, in 45 ml Dimethylacetamid gelöst, werden nach Zugabe von 21 g Bariumhydroxyd $[Ba(OH)_2 \cdot 8\ H_2O)$ und 13 ml Dimethylsulfat wie unter Beispiel 5 beschrieben umgesetzt und aufgearbeitet. Das Rohprodukt (6.1 g) wird zur Abspaltung der Isopropylidengruppe in 360 ml Eisessig-Wasser 1:1 gelöst, 16 Stunden bei Raumtemperatur stehen gelassen, mit 1.5 Liter Wasser verdünnt, mit Chloroform ausgeschüttelt und im Vakuum eingeengt. Das rohe 3′-Methyldigitoxigenin-bis-digitoxosid wird mit Cyclohexan-Essigester 3:1 über eine Cellulosesäule (mit Formamid imprägniert) gereinigt. Die chromatographisch einheitlichen Fraktionen liefern nach Kristallisation aus Essigester 1.1 g 3′-Methyl-digitoxigenin-bis-digitoxosid.

Schmelzpunkt: 228–233°C $R_D$: 1.17

Beispiel 8
3′, 9′-Dimethyl-digitoxigenin-bis-digitoxosid
5 g Isopropyliden-digitoxin, in 37 ml Dimethylacetamid gelöst, werden nach Zugabe von 17 g Bariumhydroxyd $[Ba(OH)_2 \cdot 8\ H_2O)$ und 11 ml Dimethylsulfat 1.5 Stunden bei Raumtemperatur gerührt und wie unter Beispiel 5 beschrieben aufgearbeitet. Das Rohprodukt (3.4 g) wird zur Abspaltung der Isopropylidengruppe in 210 ml Eisessig-Wasser 1:1 gelöst, 16 Stunden bei Raumtemperatur stehen gelassen, mit 750 ml Wasser verdünnt, mit Chloroform ausgeschüttelt und im Vakuum eingeengt. Das rohe 3′, 9′-Dimethyldigitoxin wird mit Cyclohexan-Essigester 4:1 über eine Cellulosesäule (mit Formamid imprägniert) gereinigt. Die chromatographisch einheitlichen Fraktionen liefern 700 mg reines 3′, 9′-Dimethyldigitoxin.

Zur Abspaltung der endstädnigen Digitoxose wird, in 3.7 ml Chloroform und 11 ml Methanol gelöst, unter Rühren bei Raumtemperatur innerhalb 30 Minuten tropfenweise mit 2.7 ml 10proz. wässriger Natriumperjodatlösung versetzt, 1.5 Stunden bei Raumtemperatur geührt, filtriert, mit Wasser verdünnt, mit Chloroform ausgeschüttelt, die Chloroformphasen mit Wasser gewaschen, über Natriumsulfat getrocknet und im Vakuum eingeengt. Das Rohprodukt wird, in 15 ml 95proz. Methanol gelöst, nach Zugabe von 75 mg Natriumborhydrid 1 Stunde bei Raumtermperatur gerührt, mit Chloroform ausgeschüttelt, die Chloroformphasen mit Wasser gewaschen, über Natriumsulfat getrocknet und im Vakuum eingeengt. Der Rückstand (0.6 g) wird in 15 ml Methanol gelöst, mit 2.7 ml 0.05 N Salzsäure 3 Stunden bei Raumtemperatur gerührt, mit 5proz. wässriger Natriumbicarbonatlösung neutralisiert, mit 15 ml Wasser verdünnt, mit Chloroform ausgeschüttelt, die Chloroformphasen mit Wasser

gewaschen, über Natriumsulfat getrocknet und im Vakuum eingeengt. Der Rückstand, aus Chloroform-Ether-Petrolether kristallisiert, liefert 520 mg 3′, 9′-Dimethyl-digitoxigenin-bis-digitoxosid.

Schmelzpunkt: 111–115°C $R_D$: 1.25

Beispiel 9
9′-Methyl-digoxigenin-bis-digitoxosid
4 g Aluminiumsopropylat in 20 ml Dimethylformamid werden mit 3.2 ml Methyljodid und 4 g Digoxigenin-bis-digitoxosid wie unter Beispiel 1 beschrieben umgesetzt und aufgearbeitet. Das Rohprodukt wird in Chloroform gelöst über 30 g Aluminiumoxyd (Merck, nach Brockmann) mit Chloroform, Chloroform-Methanol 1:1 fraktioniert. Die Chloroformfraktion liefert nach Kristallisation aus Ethanol-Wasser und Essigester 2.34 g 9′-Methyl-digoxigenin-bis- digitoxosid.

Schmelzpunkt: 223–226°C $R_{DG}$: 2.62

Beispiel 10
10′-Methyl-digoxigenin-bis-digitoxosid
2 g Digoxigenin-bis-digitoxosid, in 14 ml Dimethylformamid und 14 ml Toluol gelöst, werden wie unter Beispiel 2 beschrieben umgesetzt und aufgearbeitet. Das Rohprodukt wird einer multiplikativenVerteilung mit demPhasengemischChloroform-Tetrachlorkohlenstoff-Methanol-Wasser 1:1:1:1 unterworfen. Die eingeengte organische Phase kommt zur multiplikativen Verteilung mit demPhasengemischTetrachlorkohlenstoff-Essigester-Methanol-Wasser 3:1:2:2. Aus der wässrigen Phase erhält man nach Ausschütteln mit Chloroform, Einengen im Vakuum und Kristallisation aus Methanol-Wasser 1.48 g 10′-Methyl-digoxigenin-bis-digitoxosid.

Schmelzpunkt: 154–157°C $R_{DG}$: 2.63

Beispiel 11
10′-Ethyl-digoxigenin-bis-digitoxosid
2 g Digoxigenin-bis-digitoxosid, in 16 ml Dimethylformamid gelöst, werden wie unter Beispiel 3 beschrieben umgesetzt und aufgearbeitet. Das Rohprodukt wird mit Cyclohexan-Essigester 1:1 über eine Cellulosesäule (mit Formamid imprägniert) getrennt. Die chromatogrophisch einheitlichen Fraktionen liefern nach Kristallisation aus Aceton-Ether 860 mg 10′-Ethyl-digoxigenin-bis-digitoxosid.

Schmelzpunkt: 232–236°C $R_{DG}$: 3.12

Beispiel 12
10′-n-Propyl-digoxigenin-bis-digitoxosid
2 g Digoxigenin-bis-digitoxosid, in 16 ml Dimethylformamid gelöst, werden mit 1.16 g Bariumhydroxyd, 392 mg Bariumoxyd und 1.68 ml Di-n-propylsulfat wie unter Beispiel 3 beschrieben umgesetzt und aufgearbeitet. Das Rohprodukt wird mit Cyclohexan-Essigester 2:1 über eine Cellulosesäule (mit Formamid imprägniert) getrennt. Die chromatographisch einheitlichen Fraktionen ergeben nach Kristallisation aus Aceton-Ether 760 mg 10′-n-Propyldigoxigenin-bis-digitoxosid.

Schmelzpunkt: 248–251°C $R_{DG}$: 3.62

Beispiel 13
9′, 10′-Dimethyl-digoxigenin-bis-digitoxosid
4 g Digoxigenin-bis-digitoxosid, in 48 ml Dimethylacetamid gelöst, werden wie unter Beispiel 5 beschrieben umgesetzt und aufgearbeitet. Das Rohprodukt wird mit Cyclohexan-Essigester 3:1 über eine Cellulosesäule (mit Formamid imprägniert) getrennt. Die chromatographisch einheitlichen Fraktionen liefern nach Kristallisation aus Aceton 1.74 g 9′, 10′-Dimethyl-digoxigenin-bis-digitoxosid.

Schmelzpunkt: 133–137°C $R_{DG}$: 3.62

Beispiel 14
3′, 9′, 10′-Trimethyl-digoxigenin-bis-digitoxosid
3 g Digoxigenin-bis-digitoxosid, in 36 ml Dimethylacetamid gelöst, werden nach Zugabe von 8.4 g Bariumhydroxyd [$Ba(OH)_2 \cdot 8\,H_2O$] und 10.8 ml Dimethylsulfat wie unter Beispiel 5 beschrieben umgesetzt und aufgearbeitet. Das Rohprodukt wird mit Cyclohexan-Essigester 3:1 über eine Cellulosesäule (mit Formamid imprägniert) getrennt. Die chromatographisch einheitlichen Fraktionen ergeben nach Kristallisation aus Aceton-Ether-Petrolether 920 mg 3′, 9′, 10′-Trimethyl-digoxigenin-bis-digitoxosid.

Schmelzpunkt: 120–122°C $R_{DG}$: 4.12

Beispiel 15
9′-Acetyl-10′-methyl-digoxigenin-bis-digitoxosid
2 g 10′-Methyl-digoxigenin-bis-digitoxosid, in 20 ml Dimethylformamid gelöst, werden nach Zugabe von 400 mg Triethylendiamin und 0,4 ml Essigsäureanhydrid 24 Std. bei Raumtemperatur stehen gelassen. Anschliessend wird mit 200 ml Wasser verdünnt, mit Chloroform ausgeschüttelt und die Chloroformphasen, nach Waschen mit 2 N-Schwefelsäure, Sodalösung und Wasser, im Vacuum eingeengt. Das Rohprodukt wird mit Cyclohexan-Essigester 2:1 über eine Cellulosesäule (mit Formamid imprägniert) getrennt. Die chromatographisch einheitlichen Fraktionen ergeben nach Kristallisation aus Ether-Petrolether 530 mg 9′-Acetyl-10′-methyl-digoxigenin-bis-digitoxosid.

Schmelzpunkt: 141–145°C $R_{DG}$: 4.20

Beispiel 16
12-Acetyl-10′-methyl-digoxigenin-bis-digitoxosid
4 g 10′-Methyl-digoxigenin-bis-digitoxosid, in 20 ml Pyridin gelöst, werden nach Zugabe von 520 mg Essigsäureanhydrid 24 Std. bei Raumtemperatur stehen gelassen, mit Wasser verdünnt, mit Chloroform ausgeschüttelt und die

Chloroformphasen nach Waschen mit 2 N-Schwefelsäure und Wasser im Vacuum eingeengt. Das Rohprodukt wird mit Cyclohexan-Essigester 3:1 über eine Cellulosesäule (mit Formamid imprägniert) fraktioniert. Die chromatographisch einheitlichen Fraktionen liefern nach Kristallisation aus Aceton-Ether-Petrolether 1,43 g 12-Acetyl-10′-methyl-digoxigenin-bis-digitoxosid.

Schmelzpunkt: 204–208°C $R_{DG}$:4.29

Beispiel 17
3′, 9′-Diacetyl-10′-methyl-digitoxigenin-bis-digitoxosid
1 g 10′-Methyl-digitoxigenin-bis-digitoxosid, in 10 ml Pyridin gelöst, wird nach Zugabe von 5 ml Essigsäureanhydrid und 100 mg 4-Dimethylaminopyridin 2 Std. bei Raumtemperatur stehen gelassen, mit Wasser verdünnt, mit Chloroform ausgeschüttelt und die Chloroformphasen nach Waschen mit 2 N-Schwefelsäure und Wasser über Aluminiumoxyd filtriert und im Vacuum eingeengt. Das Rohprodukt liefert nach Kristallisation aus Ether-Petrolether 960 mg 3′, 9′-Diacetyl-10′-methyl-digitoxigenin-bis-digitoxosid.

Schmelzpunkt: 116–120°C $R_{DG}$:1.40

Beispiel 18 (Tabletten)

| | | |
|---|---|---|
| I | 10′-Methyldigitoxigenin-bis-digitoxosid | 0,050–0,250 Gew.-Teile |
| | Lactose | 45,450–45,250 Gew.-Teile |
| | Maisstärke | 10,000 Gew.-Teile |
| II | Polyvinylpyrrolidon | 2,000 Gew.-Teile |
| III | Natriumcarboxymethylstärke | 2,000 Gew.-Teile |
| | Mikrokristalline Cellulose | 15,000 Gew.-Teile |
| | Magnesiumstearat | 0,500 Gew.-Teile |

Herstellung: Eine Mischung von I wird mit einer wässrigen Lösung von II granuliert, getrocknet und gesiebt. Das Granulat wird mit den Substanzen unter III vermischt. Die Tablettenmasse wird zu Tabletten von 75 mg tablettiert.

Beispiel 19 (Liquidum)

| | |
|---|---|
| 9′,10′-Dimethyl-digitoxigenin-bis-digitoxosid | 7,5 mg |
| Glycerin | 3,0 ml |
| Ethanol | ad 10,0 ml |

Herstellung: Der Wirkstoff wird in einer Teilmenge des Ethanols gelöst. Der Lösung wird Glycerin zugesetzt. Die Auffüllung bis zum Endvolumen erfolgt mit Ethanol.

Beispiel 20 (Ampullen)

| | | |
|---|---|---|
| I | 9′-Acetyl-10′-methyldigoxigenin-bis-digitoxosid | 0,050–0,250 mg |
| | Ethanol | 90,0 mg |
| II | Propylenglykol | 400,0 mg |
| | Wasser | ad 1,0 ml |

Herstellung: Aus I und II werden getrennte Lösungen hergestellt. Beide Lösungen werden zusammen gemischt und mit Wasser zum gewünschten Volumen aufgefüllt.

**Patentansprüche**

1. Cardenolid-bis-digitoxoside der Formel I

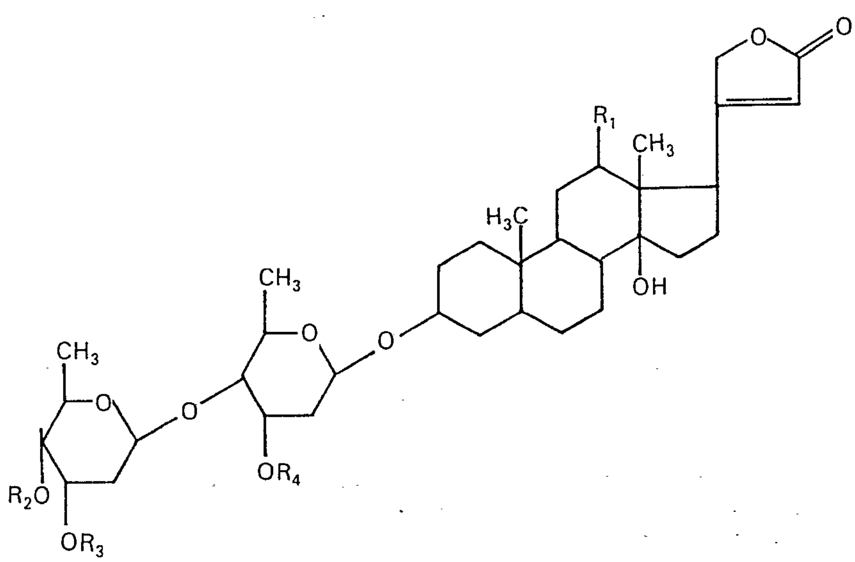

(I)

in der
$R_1$ Wasserstoff oder eine Hydroxylgruppe oder einen Acyloxyrest mit 1–3 C-Atomen darstellt,
$R_2$, $R_3$ und $R_4$ gleich oder verschieden sind und Wasserstoff, Acylgruppen mit 1–3 Kohlenstoffatomen oder Alkylgruppen mit 1–3 Kohlenstoffatomen bedeuten,
wobei die Verbindung jedoch mindestens eine Alkylgruppe enthält.

2. Verfahren zur Herstellung von Cardenolid-bis-digitoxosiden der Formel I

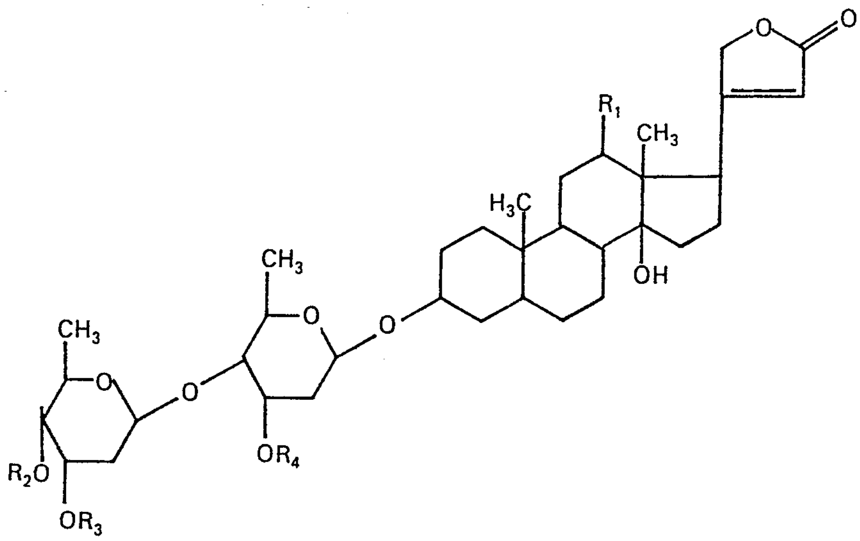

(I)

in der
$R_1$ Wasserstoff oder eine Hydroxylgrupe oder einen Acyloxyrest mit 1-3 C-Atomen darstellt,
$R_2$, $R_3$ und $R_4$ gleich oder verschieden sind und Wasserstoff, Acylgruppen mit 1-3 Kohlenstoffatomen oder Alkylgruppen mit 1-3 Kohlenstoffatomen bedeuten,
wobei die Verbindung jedoch mindestens eine Alkylgruppe enthält,
dadurch gekennzeichnet, dass man

a) Verbindungen der Formel II

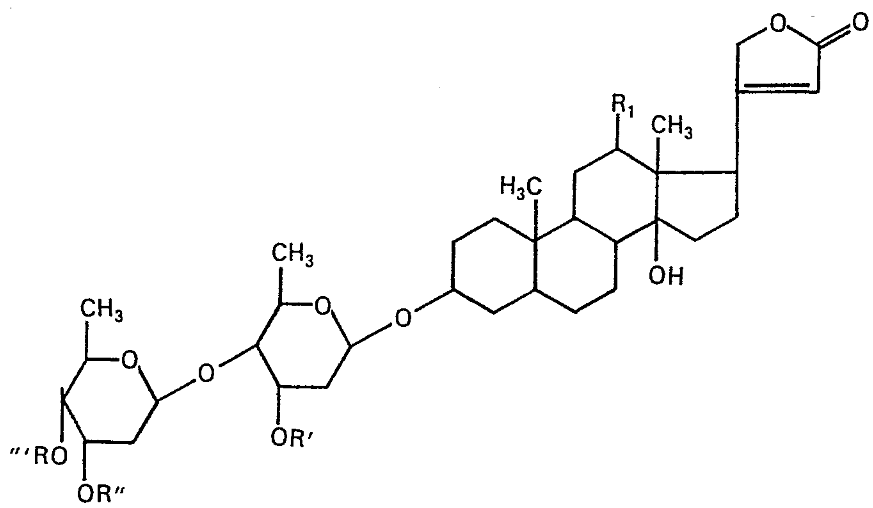

(II)

in der
$R_1$ Wasserstoff oder eine Hydroxylgruppe oder einen Acyloxyrest
R′ Wasserstoff, eine Acylgruppe oder eine Alkylgruppe und
R″ und R‴ Wasserstoff, eine Alkylgruppe, eine Acylgruppe oder zusammen ein cyclisches Acetal oder Ketal bedeuten, wobei zumindest eine der Gruppen R′, R″ und R‴ Wasserstoff ist,

in an sich bekannter Weise mit einem geeigneten Alkylierungsmittel ein- oder mehrfach alkyliert, worauf gegebenenfalls eine Acyl-, Acetal- oder Ketalgruppe durch selektive Hydrolyse abgespalten wird, worauf gegebenenfalls eine freie Hydroxylgruppe mit einem geeigneten Acylierungsreagenz in eine Acyloxygruppe überführt wird, oder

b) Verbindungen der Formel III

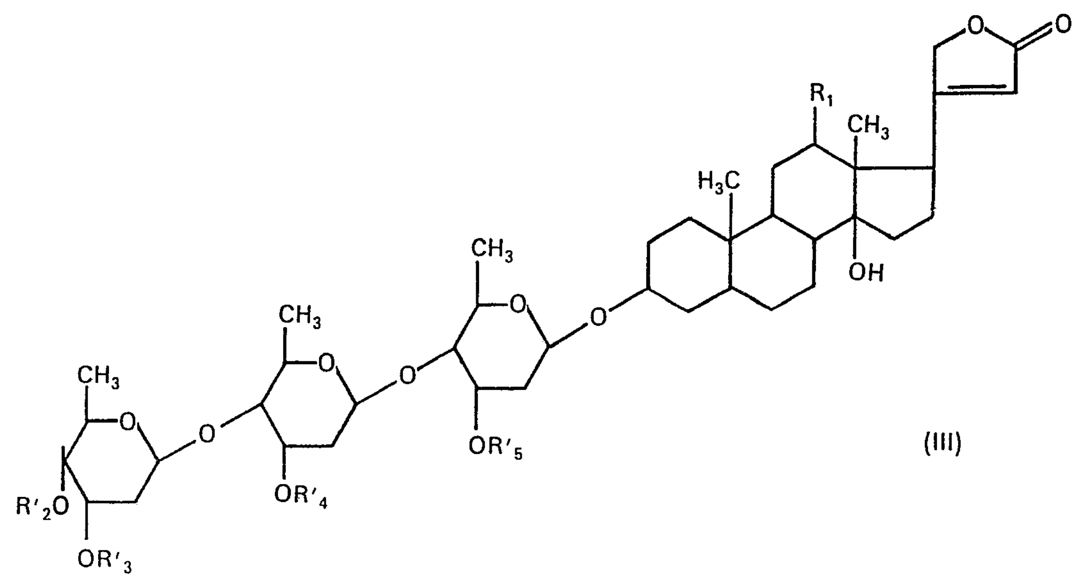

(III)

worin

$R_1$ Wasserstoff oder eine Hydroxylgruppe oder einen Acyloxyrest

$R'_2$ und $R'_3$ gemeinsam ein cyclisches Acetal oder Ketal mit 2-5 C-Atomen oder eine Acylgruppe,

$R'_4$ und $R'_5$ Alkylgruppen mit 1-3 C-Atomen oder Acylgruppen bedeuten,

durch Hydrolyse der Acyl-, Acetal- bzw. Ketalgruppe $R_2$ und $R_3$ und anschliessende oxidative Abspaltung der endständigen Digitoxose zu 3',9'-Dialkyl-digitoxigenin-bis-digitoxosid bzw. 3',9'-Dialkyl-digoxigenin-bis-digitoxosid umsetzt, worauf gegebenenfalls eine freie Hydroxylgruppe mit einem geeigneten Acylierungsreagenz in eine Acyloxygruppe überführt wird.

3. Cardenolid-bis-digitoxosiden gemäss Anspruch 1 zur Verwendung bei der Bekämpfung der Herzinsuffizienz.

4. Arzneimittel enthaltend eine Verbindung gemäss Anspruch 1 sowie geeignete pharmakologisch verträgliche Hilfs- und Trägerstoffe.

5. 9'-Methyl-digitoxigenin-bis-digitoxosid.

## Revendications

1. Cardénolide-bis-digitoxosides de formule I

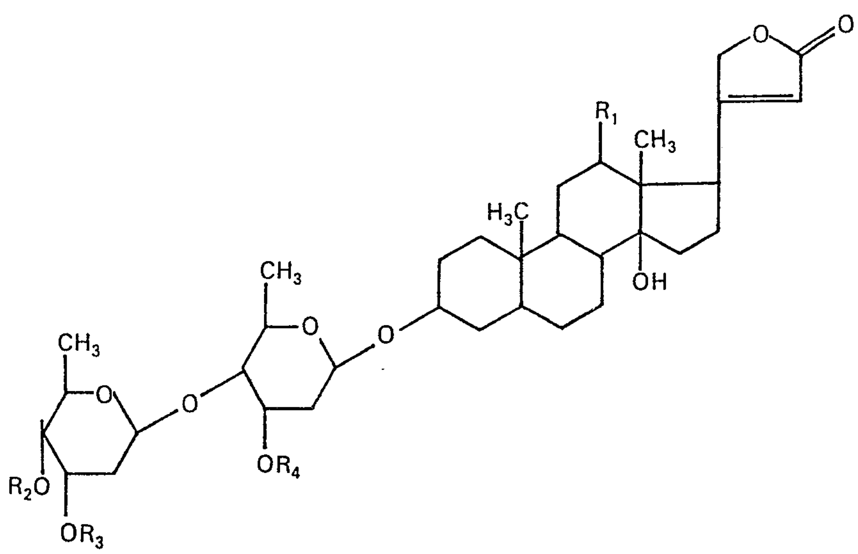

(I)

dans laquelle

$R_1$ est de l'hydrogène ou un groupe hydroxyle ou un reste acyloxy ayant de 1 à 3 atomes de carbone,

$R_2$, $R_3$ et $R_4$ sont identiques ou différents et sont de l'hydrogène, des groupes acyle ayant de 1 à 3 atomes de carbone ou des groupes alkyle ayant de 1 à 3 atomes de carbone,

le composé renfermant toutefois au moins un groupe alkyle.

2. Procédé de préparation de cardénolide-bis-digitoxosides de formule I

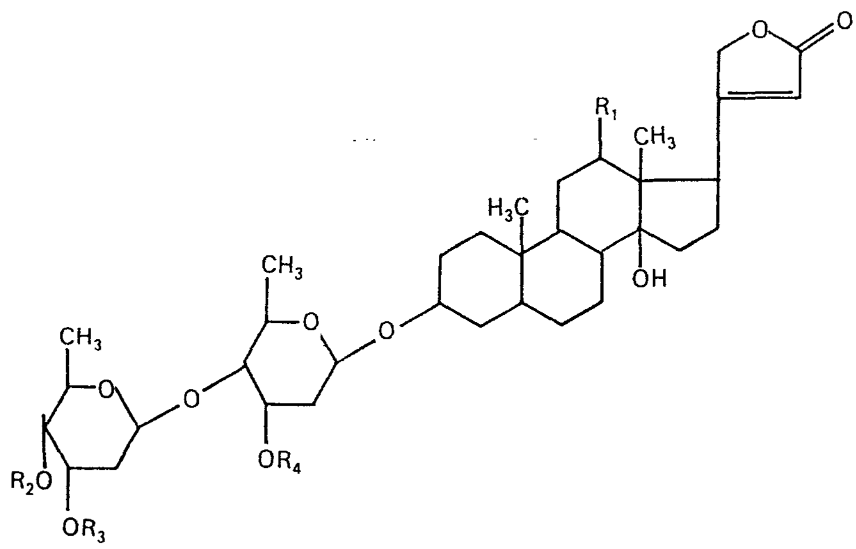

(I)

dans laquelle
$R_1$ est de l'hydrogène ou un groupe hydroxyle ou un reste acyloxy ayant de 1 à 3 atomes de carbone,
$R_2$, $R_3$ et $R_4$ sont identiques ou différents et sont de l'hydrogène, des groupes acyle ayant de 1 à 3 atomes de carbone ou des groupes alkyle ayant de 1 à 3 atomes de carbone,
le composé renfermant toutefois au moins un groupe alkyle, caractérisé en ce que l'on fait réagir
a) des composés de formule II

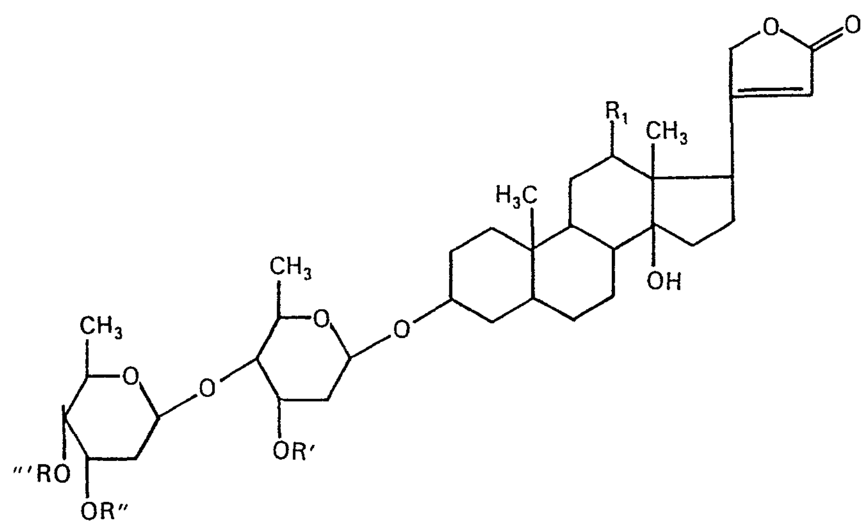

(II)

dans laquelle:
$R_1$ est de l'hydrogène ou un groupe hydroxyle ou un reste acyloxy
R′ est de l'hydrogène, un groupe acyle ou un groupe alkyle et
R″ et R‴ sont de l'hydrogène, un groupe alkyle, un groupe acyle ou forment ensemble un acétal ou un cétal cyclique, un des groupes R′, R″ et R‴ au moins étant un atome d'hydrogène,
de façon en soi connue par alkylation simple ou multiple au moyen d'un agent d'alkylation approprié, ensuite on élimine un groupe acyle, acétal ou cétal par hydrolyse sélective, après quoi on transforme éventuellement un groupe hydroxyle libre en un groupe acyloxy au moyen d'un agent d'acylation approprié, ou

b) des composés de formule III

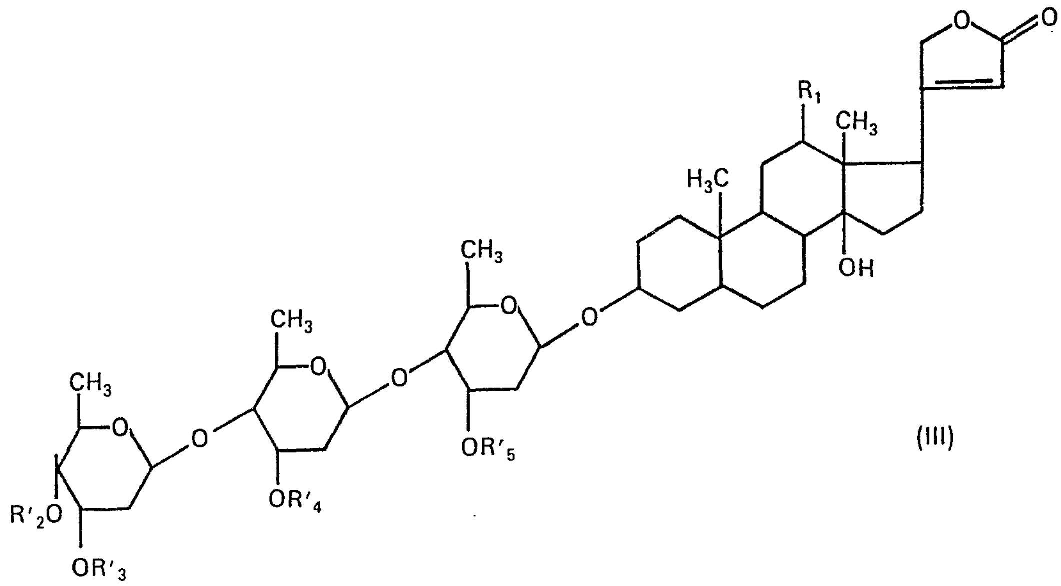

(III)

dans laquelle:
$R_1$ est de l'hydrogène ou un groupe hydroxyle ou un reste acyloxy
$R'_2$ et $R'_3$ forment ensemble un acétal ou un cétal cyclique ayant 2 à 5 atomes de carbone, ou un groupe acyle,
$R'_4$ et $R'_5$ sont des groupes alkyle ayant de 1 à 3 atomes de carbone, ou des groupes acyle,
par hydrolyse du groupe acyle, acétal ou cétal $R_2$ et $R_3$, suivie par l'élimination oxydative du digitoxose terminal pour obtenir le 3',9'-dialkyl-digitoxigénine-bis-digitoxoside ou le 3', 9'-dialkyl-digoxigénine-bis-digitoxoside, après quoi on transforme éventuellement un groupe hydroxyle libre au moyen d'un agent d'acylation en un groupe acyloxy.

3. Utilisation des cardénolide-bis-digitoxosides selon la revendication 1 dans la lutte contre l'insuffisance cardiaque.

4. Médicament renfermant un composé selon la revendication 1 ainsi que des substances auxiliaires et de support appropirées pharmacologiquement tolérables.

5. Le 9'-méthyl-digitoxigénine-bis-digitoxoside.

## Claims

1. Cardenolide-bis-digitoxosides of the formula I

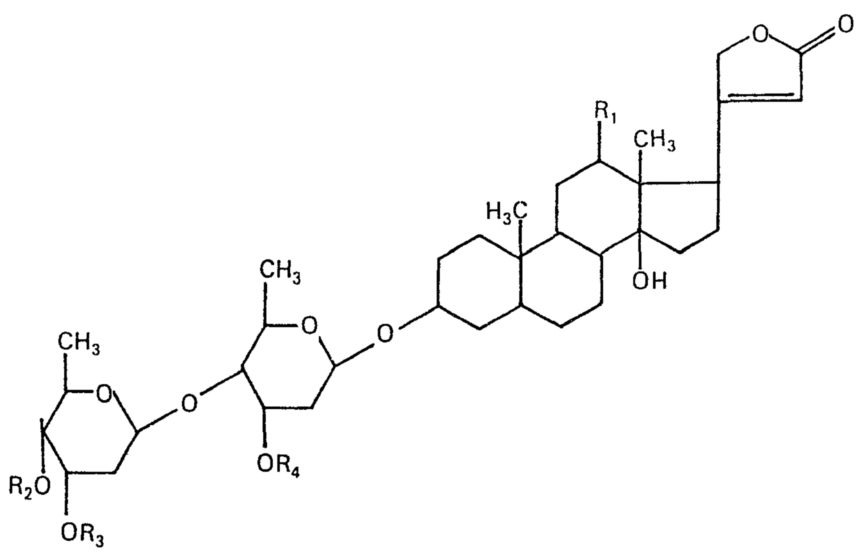

(I)

in which $R_1$ signifies hydrogen or a hydroxyl group or an acyloxy radical with 1-3 C-atoms, $R_2$, $R_3$ and $R_4$ are the same or different and signify hydrogen, acyl groups with 1-3 carbon atoms or alkyl groups with 1-3 carbon atoms, whereby, however, the compound contains at least one alkyl group.

2. Process for the preparation of cardenolide-bis-digitoxosides of the formula I

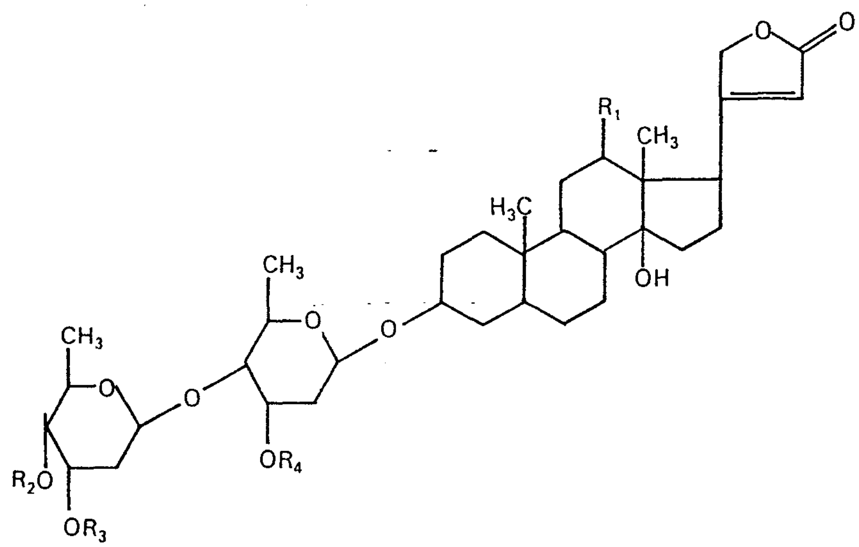

(I)

in which $R_1$ signifies hydrogen or a hydroxyl group or an acyloxy radical with 1-3 C-atoms, $R_2$, $R_3$ and $R_4$ are the same or different and signify hydrogen, acyl groups with 1-3 carbon atoms or alkyl groups with 1-3 carbon atoms, whereby, however, the compound contains at least one alkyl group, characterised in that one

a) in per se known manner one alkylates one or more times, with a suitable alkylation agent, compounds of the formula II

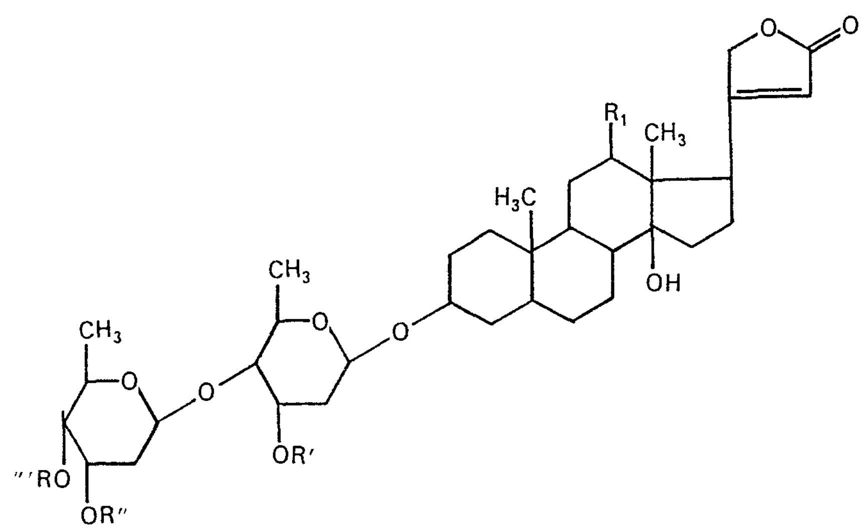

(II)

in which $R_1$ signifies hydrogen or a hydroxyl group or an acyloxy radical, R′ hydrogen, an acyl group or an alkyl group and R″ and R‴ hydrogen, an alkyl group, an acyl group or together a cyclic acetal or ketal, whereby at least one of the groups R′, R″ and R‴ is hydrogen, whereupon an acyl, acetal or ketal group is possibly split off by selective hydrolysis, whereupon a free hydroxyl group is possibly converted with a suitable acylation agent into an acyloxy group, or

b) one reacts compounds of the formula III

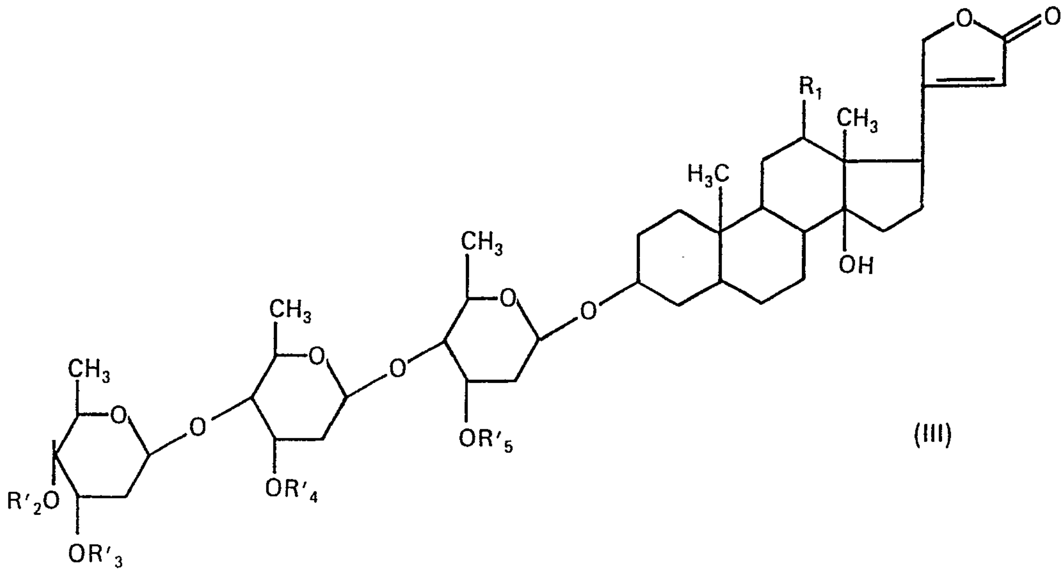

(III)

in which $R_1$ signifies hydrogen or a hydroxyl group or an acyloxy radical, $R'_2$ and $R'_3$ together a cyclic acetal or ketal with 2-5 C-atoms or an acyl group, $R'_4$ and $R'_5$ alkyl groups with 1-3 C-atoms or acyl groups, by hydrolysis of the acyl, acetyl or ketal group $R_2$ and $R_3$ and subsequent oxidative splitting off of the terminal digitoxose to give 3',9'-dialkyldigitoxigenin-bis-digitoxoside or 3'-9'-dialkyldigoxigenin-bis-digitoxoside, whereupon a free hydroxyl group is possibly converted with a suitable acylation agent into an acyloxy group.

3. Cardenolide-bis-digitoxosides according to claim 1 for use in the combating of heart insufficiency.

4. Medicaments containing a compound according to claim 1, as well as suitable pharmacologically compatible adjuvant and carrier materials.

5. 9'-Methyl-digitoxigenin-bis-digitoxoside.